(19) Europäisches Patentamt
European Patent Office
Office européen des brevets

(11) Veröffentlichungsnummer: **0 137 177**
**B1**

(12) **EUROPÄISCHE PATENTSCHRIFT**

(45) Veröffentlichungstag der Patentschrift: **28.10.87**

(51) Int. Cl.⁴: **C 07 D 401/04,** C 07 D 405/04, C 07 D 413/04, C 07 D 417/04, C 12 Q 1/34, C 12 Q 1/44

(21) Anmeldenummer: **84109115.0**

(22) Anmeldetag: **01.08.84**

(54) **Chromogene und fluorogene Carbonsäureester, Verfahren zu deren Herstellung sowie Verfahren und Mittel zum Nachweis und zur Bestimmung von Hydrolasen.**

(30) Priorität: **13.08.83 DE 3329394**

(43) Veröffentlichungstag der Anmeldung: **17.04.85 Patentblatt 85/16**

(45) Bekanntmachung des Hinweises auf die Patenterteilung: **28.10.87 Patentblatt 87/44**

(84) Benannte Vertragsstaaten: **CH DE FR GB IT LI NL SE**

(56) Entgegenhaltungen:
**EP - A - 0 039 880**
**EP - A - 0 050 877**
**DE - A - 2 950 291**

**CHEMICAL ABSTRACTS, Vol. 88, No. 4, 23. Jänner 1978, Columbus, Ohio, USA. NAIK, H.A.; SESHADRI, S.: "Studies in the Vilsmeier-Haack reaction: Part XVI. Synthesis of 7-amino-3-hetrarylquinoline fluorophore and derivatives", Seite 60, Spalte 1, Abstract-No. 24241x**
**CHEMICAL ABSTRACTS, Vol. 90, No. 7, 12. Februar 1979, Columbus, Ohio, USA. PETERS, JOSEPHINE; NASH, H.R.: "Esterases of Mus musculus: substrate and inhibition characteristics, new isozymes, and homologies with man". Seite 387, Spalte 2, Abstract-No. 52263r**

(56) Entgegenhaltungen: (Fortsetzung)
**CHEMICAL ABSTRACTS, Vol. 91, No. 23, 3. Dezember 1979, Columbus, Ohio, USA. BURGANOV, R.K.; ISHMURATOVA, R.A.; KADOCHNIKOV, A.P.; ORLOV, KH. YA.: "Quantitative determination of chlorophos". Seite 175, Spalte 1, Abstract-No. 187945f**
**CHEMICAL ABSTRACTS, Vol. 91, No. 24, 10. Dezember 1979, Columbus, Ohio, USA. JOSEPH, K.U.; SOMAYAJULU, V.V.: "Search for fluorescent brighteners. I. Synethesis of 2-(3-coumarinyl)benzoxazoles and their fluorescence". Seite 71, Spalte 2, Abstract-No. 194611x**
**CHEMICAL ABSTRACTS, Vol. 98, No. 3, 17. Jänner 1983, Columbus, Ohio, USA. RAMASUBBU, K.; GNANGURU, K.; VENKATESAN, K.; RAMAMURTHY, V.: "Topochemical photodimerization of 7-acetoxycoumarin: the acetoxy group as a steering agent". Seite 459, Spalte 2, Abstract No. 16103v**
**CHEMICAL ABSTRACTS, Vol. 96, No. 13, 29. März 1982, Columbus, Ohio, USA. TSUCHIYA, HIRONORI; HAYASHI, TOKISHI; NARUSE, HIROSHI; TAKAGI, NOBUHIKO: "High-performance liquid chromatography of carboxylic acids using 4-bromomethyl-7-acetoxy-coumarin as fluorescence reagent", Seite 360, Spalte 2, Abstract-No. 100318k**

(73) Patentinhaber: **BAYER AG, Konzernverwaltung RP Patentabteilung, D-5090 Leverkusen 1 Bayerwerk (DE)**

(72) Erfinder: **Wolfbeis, Otto S., Dr., Im Hoffeld 32, A-8046 Graz (AT)**
Erfinder: **Harnisch, Horst, Dr., Heinenbusch 4, D-5203 Much (DE)**

**EP 0 137 177 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue farbige fluorogene Carbonsäureester, die unter dem Einfluss von Hydrolasen, speziell von Carboxylesterasen, ein stark fluoreszierendes farbiges Anion bilden und zu einer verbesserten photometrischen bzw. fluorimetrischen Bestimmungsmethode für Hydrolasen verwendet werden können.

Photometrische Methoden zur Bestimmung von Hydrolasen sind von Guilbault in «Handbook of Enzymatic Methods of Analysis» (Pergamon Press, 1973) beschrieben worden. Man unterscheidet dabei Methoden der direkten Verfolgung von enzymatischen Reaktionen durch Farbbildung von jenen, wo erst durch Zugabe eines Reagenz (z.B. Base oder nach Azokupplung) Farbstoffe gebildet werden, deren Konzentration photometrisch bestimmt wird. Als Substrate werden eingesetzt Phenyl- und Naphthylester aliphatischer Carbonsäuren, sowie Indophenolacetat. In Anal. Chim. Acta 148, 255 (1983) werden Fettsäureester des 4-(p-Sulfophenylazo)-1-naphthols als Substrate zur Lipasenbestimmung angegeben.

In entsprechender Weise, doch mit erhöhter Empfindlichkeit, lassen sich Carboxylesterasen und andere Hydrolasen fluorimetrisch mit den Fettsäureestern der folgenden Phenole bestimmen: α- und β-Naphthol, Indoxyl, N-Methylindoxyl, Fluorescein, Resorufin und Umbelliferon. In Biochem. Genet. 1978, 15 (5–6), 553–569 sind Beispiele der genannten Verbindungen beschrieben. In Anal. Biochem. 129, 365 (1983) wird ein Verfahren zur Bestimmung von verschiedenen Hydrolasen, speziell Carboxylesterasen mit Hilfe von Estern des 1-Hydroxypyren-3,6,8-trisulfonats auf fluorimetrischem oder photometrischem Weg beschrieben.

Aufgabe der vorliegenden Erfindung ist es, neue, langwellig absorbierende bzw. fluoreszierende Verbindungen zur Verfügung zu stellen, welche direkt-kinetische Enzymbestimmungen erlauben, und deren Absorption bzw. Fluoreszenz nicht durch die kurzwellige Untergrundfluoreszenz aus biologischen Flüssigkeiten gestört wird. Diese Aufgabe wird mit den erfindungsgemässen Carbonsäureestern von 7-Hydroxy-cumarin-Derivaten gelöst.

Gegenstand der Erfindung sind neue Carbonsäureester der allgemeinen Formel

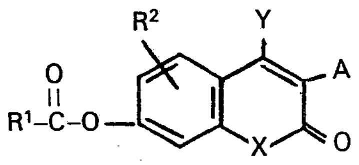

(I)

worin

$R^1$ für Wasserstoff, einen gegebenenfalls verzweigten oder cyclischen Alkyl-, Alkenyl- oder Alkinylrest mit 1 bis 20 C-Atomen, welcher gegebenenfalls 1 bis 3 Substituenten aus der Gruppe Amino, Hydroxy, Mercapto, Carboxyl und Halogen trägt und gegebenenfalls durch 1 oder 2 Heteroatome aus der Gruppe O, NQ und S unterbrochen ist, oder – zusammen mit der CO-Gruppe – für den C-terminalen Rest einer Aminosäure steht, worin $R^1$ für einen (Hetero)arylalkylrest mit 6 bis 20 C-Atomen steht, welcher gegebenenfalls 1 bis 3 Substituenten aus der Gruppe $C_1$–$C_4$-Alkyl; Halogen wie Chlor, Brom, Jod oder Fluor; $C_1$–$C_4$-Alkoxy, Amino, Hydroxy, Mercapto, Carboxyl oder Sulfosäure trägt, oder – zusammen mit der CO-Gruppe – für das C-terminale Ende eines Peptids aus 2 bis 10 Aminosäuren steht,

X –O– oder –NQ– darstellt,

Q für Wasserstoff, einen gegebenenfalls durch 1–2 OH-Gruppen substituierten $C_1$–$C_4$-Alkylrest oder $C_1$–$C_4$-Alkoxycarbonylrest,

Y für Wasserstoff, Halogen (bevorzugt Chlor oder Brom), Cyano oder einen Carbamoylrest,

$R^2$ für Wasserstoff oder eine Sulfonsäuregruppe und

A für einen gegebenenfalls durch 1–2 Substituenten aus der Gruppe $C_1$–$C_4$-Alkyl, Chlor, $C_1$–$C_4$-Alkoxy, $C_1$–$C_4$-Alkylsulfonyl, Carboxyl, $C_1$–$C_4$-Alkoxycarbonyl, Cyclohexyl, Phenyl oder Sulfonsäure substituierten Benzoxazol-2-yl, Benzthiazol-2-yl, Thiazol-5-yl, Benzimidazol-2-yl, Chinazol-4-on-2-yl, 5-Phenyl-1,3,4-thiadiazol-2-yl oder 4-Pyridyl-Rest steht.

Wenn $R^1CO$ für den C-terminalen Rest einer Aminosäure oder eines Peptids steht, dann können die in $R^1$ vorhandenen Aminogruppen selbstverständlich gegebenenfalls durch aus der Proteinchemie an sich bekannte Schutzgruppen (z.B. Formyl-, Acetyl-, Butoxycarbonyl-, Carbobenzyloxycarbonyl- oder Tosylrest) blockiert sein.

Bevorzugt stellt $R^1$ in Formel (I) eine unverzweigte $C_1$–$C_{18}$-Alkyl- oder -Alkylengruppe oder – zusammen mit der CO-Gruppe – den C-terminalen Rest einer natürlichen Aminosäure oder eines Peptids aus 2 bis 5 Aminosäuren dar.

Besonders bevorzugt steht $R^1$ für Methyl, Propyl oder – zusammen mit der CO-Gruppe – für den Rest einer natürlichen Fettsäure, einer der im Säugetierorganismus vorkommenden linksdrehenden Aminosäuren (z.B. Glycin, Alanin, β-Phenylalanin, Leucin, Histidin, Arginin oder eines Peptids bestehend aus 2 oder 3 linksdrehenden Aminosäuren.

Beispiele für besonders bevorzugte Reste $R^1CO$ sind Acetyl, Butyryl, Oleyl, N-Acetylglycyl, N-(N-Acetylphenylalanyl)-glycyl oder N-(N-Acetylalanyl)-tryptophyl.

Besonders bevorzugt sind weiterhin diejenigen Verbindungen der Formel (I), worin

A für einen gegebenenfalls durch Methyl, Ethyl, Chlor, Methoxy, Ethoxy oder Sulfo substituierten Benzthiazol-2-yl- oder Benzoxazol-2-yl-Rest und

Y für CN stehen.

Im Gegensatz zu einigen literaturbekannten Enzymsubstraten (z.B. den Derivaten des Fluoresceins und Resorufins) besitzen die erfindungsgemässen Verbindungen praktisch keine Überlappung zwischen Absorptions- und Fluoreszenzbanden (grosse Stokes-Verschiebung). Dies ist

für die Gestaltung der optischen Messanordnung von besonderem Vorteil, da handelsübliche Steilkantenfilter anstelle von teuren anderen Filtern eingesetzt werden können, und da der Streulichtanteil (aus Tyndall-, Rayleigh- und Raman-Streuung) entfällt.

Die erfindungsgemässen Carbonsäureester der Formel (I) können hergestellt werden, indem man Hydroxy-Verbindungen der Formel

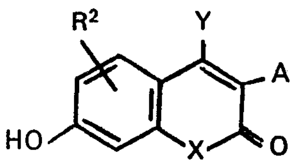

(II)

worin

X, A, Y und $R^2$ die oben genannte Bedeutung besitzen, mit einem Säureanhydrid oder Säurehalogenid, letztere vorzugsweise in Gegenwart eines anorganischen oder organischen Säurefängers, in an sich bekannter Weise umsetzt.

Als Säurefänger eignen sich anorganische und organische Basen, beispielsweise wasserfreies Kalium- oder Natriumcarbonat, Magnesiumoxid, Triethylamin, Kollidin, Picolingemische, Pyridin und Dimethylanilin.

Die Reaktion wird gegebenenfalls in Gegenwart eines Lösungs- oder Verdünnungsmittels, vorzugsweise im Temperaturbereich von 0 bis 100 °C, durchgeführt.

Als Lösungs- und Verdünnungsmittel eignen sich unter den Reaktionsbedingungen inerte organische Flüssigkeiten wie Toluol, Chlorbenzol, Dichlorbenzol, Dichlormethan, Chloroform, Tetrachlormethan, Dichlorethan, Trichlorethylen, Acetonitril, Dioxan, Tetrahydrofuran oder eine der oben genannten organischen Basen wie Pyridin.

Eine weitere Möglichkeit zur Herstellung von Verbindungen der Formel (I) besteht darin, dass man Aldehyde der Formel

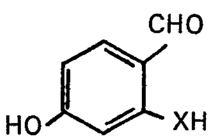

(III),

worin

X die oben angegebene Bedeutung besitzt, mit Verbindungen der Formel

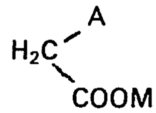

(IV),

worin

A die oben genannte Bedeutung besitzt und

M für Wasserstoff oder ein Alkalimetall steht, in einem Anhydrid der Formel

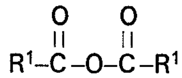

(V),

worin

$R^1$ die oben angegebene Bedeutung hat, vorzugsweise jedoch für Methyl oder Propyl steht, als Lösungs- oder Kondensationsmittel in Gegenwart eines Alkalisalzes der entsprechenden Carbonsäure

$$R^1\text{–}COOH$$

unter den Bedingungen der Perkin-Oglialoro-Synthese (J.R. Johnson, Org. React. I 210 (1942) ) durch mehrstündiges Erhitzen auf Temperaturen von 100–140 °C umsetzt.

Dieses Verfahren bietet sich immer dann als vorteilhaft an, wenn Verbindungen der Formel (IV), wie Pyridyl-4-essigsäure, gut zugänglich sind.

Neben diesen beiden üblichen Verfahren zur Herstellung von Carbonsäurearylestern sind auch andere Methoden zur Estersynthese geeignet, wie sie z.B. von H. Henecka in Houben-Weyl, Methoden der organischen Chemie, Band 8. Teil 3, Seite 508 beschrieben sind. Erwähnt werden soll auch die Synthese langkettiger Fettsäureester mit Hilfe von Carbonsäuren durch vorherige Aktivierung mit Dicyclohexylcarbodiimid.

Es versteht sich von selbst, dass Aminosäuren wie Alanin zuvor mit einer aus der Peptidchemie bekannten Schutzgruppe, z.B. dem Tosylrest, geschützt werden müssen.

Die Ausgangsverbindungen der Formel (II) sind bekannt (US-PS 3 521 128; DE-OS 21 00 295 und DE-OS 3 229 301) oder nach den dort genannten Methoden herstellbar.

Die neuen Verbindungen der Formel (I) sind zum Teil kaum wasserlösliche, schwach blau (Y=H, Cl, Br) oder grünlich (R=CN, $CONH_2$) fluoreszierende und zumeist nur wenig farbige Substanzen, die im wässrigen Medium von Hydrolasen (speziell Carboxylesterasen) zum stark fluoreszierenden, tief gelb bis rot gefärbten mesomeren Anion der Formeln

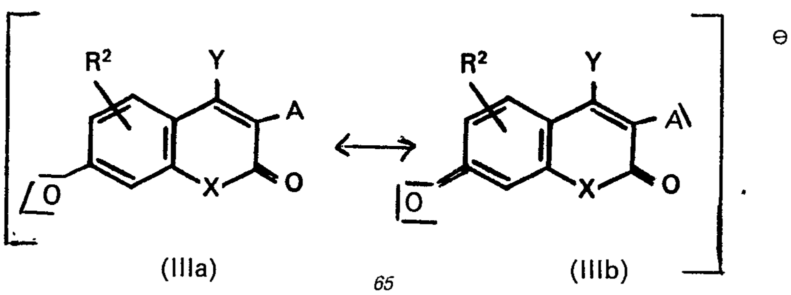

(IIIa) (IIIb)

gespalten werden.

Die neuen Reagentien der Formel (I) eignen sich daher zum direkten Hydrolase-Nachweis in biologischen Materialien. Besonders vorteilhaft können sie zur quantitativen photometrischen oder fluorimetrischen Bestimmung von Carboxylesterasen in der klinischen Analytik verwendet werden.

Durch Wahl eines geeigneten Carbonsäureesters $R^1$ lässt sich für jedes Enzym aus der Vielfalt der hydrolytisch wirksamen Enzyme (z.B. Carboxylesterase, Acetylesterase, Cholinesterasen, Lipasen, Trypsin und Chymotrypsin) ein Substrat der Formel (I) herstellen, welches bevorzugt gespalten wird.

Gegenüber den Esterase-Reagentien des Standes der Technik weisen die neuen Carbonsäureester der Formel (I) wesentliche Vorteile auf:

1. Keinerlei Überlappung der photometrisch zu verfolgenden Zunahme der Absorption des bei der enzymatischen Hydrolyse entstehenden Phenolations (IIIa–IIIb) wegen der grossen Stokes-Verschiebung gegenüber der Absorption der Carbonsäureester.

2. Keinerlei störende Überlappung der fluorimetrisch zu verfolgenden, bei der enzymatischen Spaltung von (I) entstehenden, starken Fluoreszenz des Hydroxylats (IIIa–IIIb) mit der schwachen Fluoreszenz des eingesetzten Reagenz (I) (grosse Stokes-Verschiebung).

3. Verwendung einfachster Filterfluorimeter möglich, da weder eine Anregung durch UV-Licht, bei der sich in der Regel die Untergrundfluoreszenz des biologischen Materials störend bemerkbar macht, noch die Anwendung aufwendiger Monochromatoren oder Interferenzfilter erforderlich sind, wie sie beim Einsatz von Fluorescein-carbonsäureestern gebraucht werden.

4. Bildung des tief farbigen fluoreszierenden Hydroxylatanions (IIIa–IIIb) überraschenderweise bereits unter schwach sauren Bedingungen (z.B. bei pH 6,0). Hierdurch ist es erstmalig möglich, die Aktivität von Esterasen photometrisch oder fluorimetrisch im sichtbaren Spektralbereich in kontinuierlicher Messung im schwach sauren pH-Bereich mit einer so hohen Nachweisempfindlichkeit zu verfolgen, wie sie bisher nur bei zeitaufwendigeren nichtkontinuierlichen Verfahren bekannt ist.

Die erfindungsgemässen Carbonsäureester können zur Bestimmung der Esteraseaktivität in den verschiedensten Körperflüssigkeiten (Serum; cerebrospinale Flüssigkeit; Urin) verwendet werden. Es ist jedoch auch möglich, die erfindungsgemässen Reagentien analog zur Lehre der US-PS 3 772 340 zur Messung der Bakterienkonzentration in Flüssigkeiten (z.B. bakteriell infiziertem Urin) heranzuziehen. Zu diesem Zweck werden zunächst in an sich bekannter Weise (z.B. osmotischer Schock; Sphäroplastenbildung) die bakteriellen Enzyme, darunter auch Esterasen, freigesetzt. Aus der mittels der erfindungsgemässen Verbindungen bestimmten Esteraseaktivität lassen sich dann Rückschlüsse auf die Zahl der vorhandenen Bakterien ziehen.

Bei der Bestimmung der Esterasenaktivität können die erfindungsgmässen Verbindungen wegen ihrer zum Teil schlechten Wasserlöslichkeit nur teilweise in Form wässriger Lösungen, gegebenenfalls auf den gewünschten pH-Wert gepuffert, eingesetzt werden. Gegebenenfalls muss durch Zusatz organischer Lösungsmittel wie z.B. Dioxan, Methylcellosolve, Glykol usw. die Löslichkeit in Wasser verbessert werden, wobei darauf zu achten ist, dass eine Verminderung der Enzymaktivität nicht eintritt.

Überraschenderweise werden die erfindungsgemässen Carbonsäureester deutlich besser wasserlöslich, wenn man der Lösung einen Micellarbildner in Konzentrationen über der kritischen Micellarkonzentration zusetzt. Vorzugsweise werden dabei anionische Micellarbildner verwendet, welche das durch enzymatische Hydrolyse gebildete Phenolatanion aus der Micelle in die Lösung stossen.

Eine deutlich verbesserte Wasserlöslichkeit ist bei denjenigen Verbindungen gegeben, welche im Molekül eine Sulfogruppe tragen.

Es ist jedoch auch möglich, die neuen Carbonsäureester in an sich bekannter Weise auf Träger aufzubringen und die Nachweisreaktion in heterogener Phase ablaufen zu lassen, wie sie etwa in Anal. Chem. 55, 498 A (1983) beschrieben ist.

Geeignete Trägermaterialien sind z.B. Papiere wie Filterpapier oder auf einem Kunststoff (z.B. Polystyrol) aufgebrachtes, mit einem organischen Bindemittel versehenes Siliziumdioxid. Es lassen sich auf diese Weise Teststreifen herstellen, die nach dem Aufbringen der zu untersuchenden Flüssigkeit ihre Farbe ändern bzw. zu fluoreszieren beginnen und deren Farbe bzw. Fluoreszenz mittels der in der klinischen Analytik üblichen Instrumente gemessen werden kann. Die quantitative Bestimmung der Esterase-Aktivität in einer unbekannten Probe ist möglich, wenn man den zeitlichen Verlauf der Änderung der Lichtabsorption bzw. der Fluoreszenz-Intensität in der wässrigen Lösung bzw. auf dem Teststreifen mit jenem von Standards mit bekanntem Esterasegehalt vergleicht.

Bei den im folgenden beschriebenen Versuchen wurden als Standards folgende Hydrolasenpräparate der Firma Sigma Chemical Co. verwendet:

a) Carboxylesterhydrolase(EC 3.1.1.1): Typ I (E 3128) aus Schweineleber

b) Lipase (EC 3.1.1.3): Typ VII (L 1754), aus Candida Cylindracea

c) Lipase (EC 3.1.1.3): Typ I (L 3001), aus Weizenkeimlingen

d) Acetylesterase (EC 3.1.1.6): (A 7900), aus Orangenschalen

e) Cholinesterase (EC 3.1.18): Typ X (C 0736), aus Pferdeserum

f) α-Chymotrypsin (EC 3.4.2.1.4): Typ II (C 4129) aus Rinderpankreas

Beispiel 1

Eine Suspension von 0,7 g (2,5 mMol) 3-Benzoxazolyl-7-hydroxy-cumarin und 5 ml Dimethylformamid wird mit 4 ml (4,35 g;

40 mMol) Acetanhydrid versetzt und 2 Stunden unter Ausschluss von Luftfeuchtigkeit auf 60 °C erwärmt. Beim Abkühlen scheiden sich aus der Lösung Kristalle der Verbindung der Formel

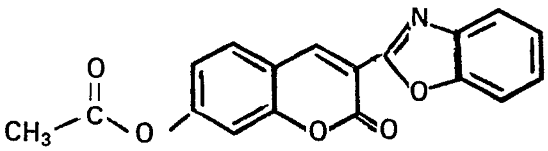

(Ausbeute: 0,6 g, das sind 85% der Theorie) ab. Die Reinigung erfolgt durch Umkristallisation aus wenig Dioxan. Man erhält leicht gelbliche Kristalle vom Schmelzpunkt 203–205 °C.

Die Verbindung ist z.B. in Aceton, Dioxan, Dimethylformamid, Dimethylsulfoxid und Ethylenglykolmonomethylether löslich.

Zur photometrischen Verfolgung der enzymatischen Hydrolyse misst man die Extinktion bei 430 nm.

Zur fluorimetrischen Verfolgung der enzymatischen Hydrolyse wählt man Anregungslicht mit einem Maximum bei 430 nm und misst die Fluoreszenz bei 470 nm. Bei dieser Wellenlänge wird ausschliesslich die Fluoreszenz des enzymatisch gespaltenen Substrats erfasst.

In analoger Weise werden unter Verwendung der entsprechenden Ausgangsverbindungen die folgenden Carbonsäureester der allgemeinen Formel

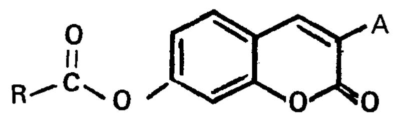

hergestellt:

| Beispiel | R | A | Schmelzpunkt |
|---|---|---|---|
| 2 | $CH_3-(CH_2)_2-$ | 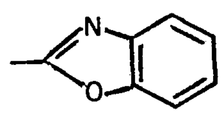 | 193–195 °C |
| 3 | $CH_3-(CH_2)_6-$ | –"– | 167–170 °C |
| 4 | $CH_3-$ | 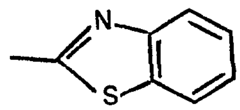 | 270–272 °C |

Beispiel 5

Zu einer Suspension von 3 g (10 mMol) 3-Benzthiazolyl-4-cyano-7-hydroxy-cumarin in 50 ml Dimethylformamid werden 10 ml (9,7 g; 60 mMol) Buttersäureanhydrid gegeben und unter Rühren und Ausschluss von Luftfeuchtigkeit für 8 Stunden auf 60 °C erwärmt. Dabei scheidet sich ein gelbes Produkt der Formel

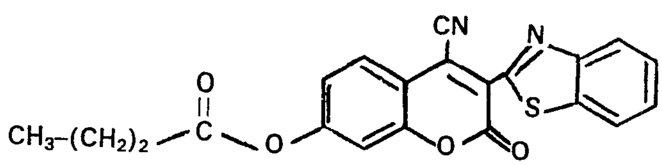

ab, das nach dem Erkalten der Reaktionsmischung abgesaugt wird (Ausbeute: 3,1 g, das sind 80% der Theorie). Zur Reinigung wird es aus Dioxan umkristallisiert und im Trockenschrank bei 110 °C getrocknet. Man erhält gelbe Kristalle vom Schmelzpunkt 244–246 °C.

Die Verbindung ist z.B. in Aceton, Dioxan und Ethylenglykolmonomethylether löslich.

Zur photometrischen Verfolgung der enzymatischen Hydrolyse misst man die Extinktion bei 510 nm.

Zur fluorimetrischen Verfolgung der enzymatischen Hydrolyse wählt man Anregungslicht mit einem Maximum bei 510 nm und misst die Fluoreszenz bei 595 nm. Bei dieser Wellenlänge wird ausschliesslich die Fluoreszenz des enzymatisch gespaltenen Substrats erfasst.

In analoger Weise werden unter Verwendung der entsprechenden Ausgangsverbindungen die folgenden Carbonsäureester der allgemeinen Formel

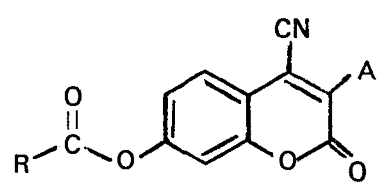

hergestellt:

| Beispiel | R | A | Schmelzpunkt |
|---|---|---|---|
| 6 | $CH_3-$ | 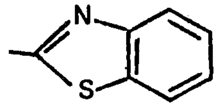 | 256–258 °C |
| 7 | $CH_3-(CH_2)_7-CH=CH(CH_2)_7-$ | –"– | 163–165 °C |

Beispiel 8

Eine Mischung von 1,1 g (2,5 mMol) 3-(5-Methyl-7-sulfobenzoxazol-2-yl)-7-hydroxy-cumarin-pyridiniumsalz, 50 ml Dimethylformamid und 4 ml (4,35 g; 40 mMol) Acetanhydrid wird unter Ausschluss von Luftfeuchtigkeit für ungefähr 4 Stunden auf 60 °C erwärmt, wobei sich eine klare Lösung bildet. Man dampft die Lösung mit Hilfe eines Rotationsverdampfers im Vakuum ein und versetzt das zurückbleibende Öl mit 20 ml Dioxan. Beim Anreiben wird das Öl kristallin und man erhält nach dem Absaugen 0,9 g (73% der Theorie) der Verbindung der Formel

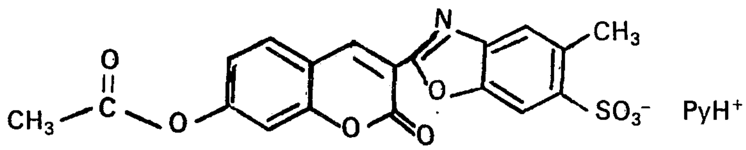

Die Reinigung erfolgt durch Umkristallisation aus Ethanol. Man erhält gelbe Kristalle, die bei 172–174 °C sintern.

Die Verbindung ist z.B. in Wasser, Methanol, Dimethylformamid und Dimethylsulfoxid löslich.

Zur photometrischen Verfolgung der enzymatischen Hydrolyse misst man die Extinktion bei 430 nm.

Zur fluorimetrischen Verfolgung der enzymatischen Hydrolyse wählt man Anregungslicht mit einem Maximum bei 430 nm und misst die Fluoreszenz bei 470 nm. Bei diesen Wellenlängen wird ausschliesslich die Fluoreszenz des enzymatisch gespaltenen Substrats erfasst.

Ein besonderer Vorteil der Anwendung dieser Verbindung als Substrat zur photometrischen und fluorimetrischen Bestimmung von Carboxylesterasen besteht in der sehr guten Wasserlöslichkeit.

Beispiel 9

In einem Photometer oder Fluorimeter füllt man bei 23 °C die Küvette mit 3 ml einer wässrigen TRIS-gepufferten (0,1 Mol TRIS*/Liter) Lösung der Verbindung des Beispiels 8 vom pH 7,4, stellt die Absorptions- bzw. Anregungswellenlänge auf 430 nm und im Fluorimeter die Emissionswellenlänge auf 470 nm ein, fügt 0,1 ml der auf Esterase-Aktivität zu bestimmenden Flüssigkeit, deren Gehalt in der Grössenordnung von 0,1 mg Esterase pro ml liegen soll, hinzu und verfolgt die zeitliche Änderung der Absorption bzw. Fluoreszenzintensität über einen Zeitraum von etwa 1–3 Minuten im Vergleich zu vorher angefertigten Eichkurven. Der anfangs lineare Anstieg der Fluoreszenzintensität ist ein direktes Mass für die Enzymaktivität.

*) TRIS = tris (hydroxymethyl)aminomethan

**Patentansprüche**

1. Verbindungen der allgemeinen Formel

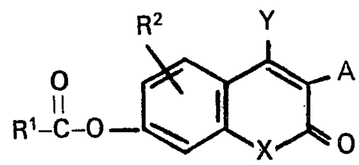

worin

$R^1$ für Wasserstoff, einen gegebenenfalls verzweigten oder cyclischen Alkyl-, Alkenyl- oder Alkinylrest mit 1 bis 20 C-Atomen, welcher gegebenenfalls 1 bis 3 Substituenten aus der Gruppe Amino, Hydroxy, Mercapto, Carboxyl und Halogen trägt und gegebenenfalls durch 1 oder 2 Heteroatome aus der Gruppe O, S und NQ unterbrochen ist, oder – zusammen mit der CO-Gruppe – für den C-terminalen Rest einer Aminosäure steht, worin $R_1$ für einen (Hetero)arylalkylrest mit 6 bis 20 C-Atomen steht, welcher gegebenenfalls 1 bis 3 Substituenten aus der Gruppe $C_1$–$C_4$-Alkyl, Halogen, $C_1$–$C_4$-Alkoxy, Amino, Hydroxy, Mercapto, Carboxyl oder Sulfonsäure trägt oder – zusammen mit der CO-Gruppe – für das C-terminale Ende eines Peptids aus 2 bis 10 Aminosäuren steht, wobei die in $R^1$ vorhandenen Aminogruppen gegebenenfalls durch an sich bekannte Schutzgruppen verschlossen sein können,

X –O– oder –NQ– darstellt,

Q für Wasserstoff, einen gegebenenfalls durch 1–2 OH-Gruppen substituierten $C_1$–$C_4$-Alkylrest oder $C_1$–$C_4$-Alkoxycarbonylrest

Y für Wasserstoff, Halogen, Cyano oder einen Carbamoylrest,

$R^2$ für Wasserstoff oder eine Sulfonsäuregruppe und

A für einen gegebenenfalls durch 1–2 Substituenten aus der Gruppe $C_1$–$C_4$-Alkyl, Chlor, $C_1$–$C_4$-Alkoxy, $C_1$–$C_4$-Alkylsulfonyl, Carboxyl, $C_1$–$C_4$-Alkoxycarbonyl, Cyclohexyl, Phenyl oder Sulfonsäure substituierten Benzoxazol-2-yl-, Benzthia-

zol-2-yl-, Thiazol-5-yl-, Benzimidazol-2-yl-, Chinazol-4-on-2-yl-, 5-Phenyl-1,3,4-thiadiazol-2-yl- oder 4-Pyridyl-Rest steht.

2. Verbindung nach Anspruch 1, dadurch gekennzeichnet, dass

$R^1$ für einen unverzweigten $C_1$–$C_{18}$-Alkyl- oder Alkenylrest steht, oder – zusammen mit der CO-Gruppe – den C-terminalen Rest einer natürlichen Aminosäure oder eines Peptids aus 2 bis 5 Aminosäuren darstellt.

3. Verbindungen nach Anspruch 1 oder 2, dadurch gekennzeichnet, dass

Y für CN und

A für einen gegebenenfalls durch Methyl, Ethyl, Chlor, Methoxy, Ethoxy oder Sulfo substituierten Benzthiazol-2-yl- oder Benzoxazol-2-yl-Rest stehen.

4. Verfahren zur Herstellung von Verbindungen nach Ansprüchen 1 bis 3, dadurch gekennzeichnet, dass man Hydroxy-Verbindungen der Formel

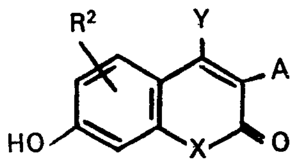

worin

X, Y, A und $R_2$ die in den Ansprüchen 1–3 genannte Bedeutung haben mit einem Säureanhydrid oder Säurehalogenid umsetzt.

5. Verfahren nach Anspruch 4, dadurch gekennzeichnet, dass man die Umsetzung mit einem Carbonsäurehalogenid in Gegenwart eines anorganischen oder organischen Säurefängers durchführt.

6. Verfahren zur Herstellung von Verbindungen gemäss Ansprüchen 1 bis 3, dadurch gekennzeichnet, dass man Aldehyde der Formel

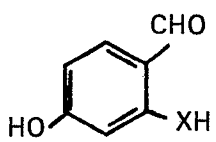

worin

X die in den Ansprüchen 1–3 genannte Bedeutung hat mit Verbindungen der Formel

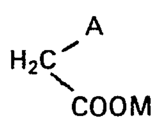

worin

A die in den Ansprüchen 1 bis 3 genannte Bedeutung hat

M für Wasserstoff oder ein Alkalimetall steht, in einem Anhydrid der Formel

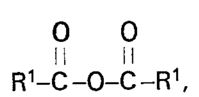

worin

$R^1$ die in den Ansprüchen 1 bis 3 genannte Bedeutung hat als Lösungs- oder Kondensationsmittel in Gegenwart eines Alkalisalzes der entsprechenden Carbonsäure

$$R^1\text{–COOH}$$

unter den Bedingungen der Perkin-Oglialoro-Synthese durch mehrstündiges Erhitzen auf Temperaturen von 100–140 °C umsetzt.

7. Chromogene bzw. fluorogene Carbonsäureester enthaltende Testmittel für den photometrischen oder fluorimetrischen Nachweis von Hydrolasen, speziell Carboxylesterasen, dadurch gekennzeichnet, dass sie als chromogene bzw. fluorogene Carbonsäureester Verbindungen nach Anspruch 1 bis 3 enthalten.

8. Testmittel nach Anspruch 7, dadurch gekennzeichnet, dass sie zur Verbesserung der Wasserlöslichkeit der Hydrolasen-Reagentien anionische oder kationische Micellarbildner enthalten.

9. Testmittel nach Anspruch 7 oder 8, dadurch gekennzeichnet, dass die Carbonsäureester in einem Trägermaterial enthalten sind.

10. Verfahren zur Bestimmung von Hydrolase-Aktivität in einer flüssigen Probe, dadurch gekennzeichnet, dass man die zu untersuchende Probe mit einer Verbindung nach Anspruch 1 bis 3 bzw. einem Testmittel nach Anspruch 7 bis 9 in Kontakt bringt und den zeitlichen Verlauf der Lichtabsorption bzw. der Fluoreszenzintensität vermisst.

**Revendications**

1. Composés de formule générale

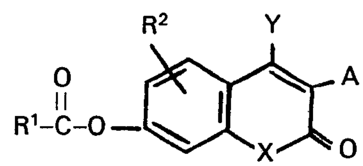

dans laquelle

$R^1$ désigne l'hydrogène, un reste alkyle, alcényle ou alcynyle éventuellement ramifié ou cyclique ayant 1 à 20 atomes de carbone, qui porte le cas échéant 1 à 3 substituants du groupe des substituants amino, hydroxy, mercapto, carboxyle et halogéno et qui est interrompu éventuellement par 1 ou 2 hétéro-atomes du groupe O, S et NQ, ou forme – conjointement avec le groupe CO – le reste d'extrémité terminale C d'un aminoacide, où $R^1$ représente un reste (hétéro)arylalkyle ayant 6 à 20 atomes de carbone, qui porte éventuellement 1 à 3 substituants du groupe des substituants alkyle en $C_1$ à $C_4$, halogéno, alkoxy en $C_1$ à $C_4$, amino, hydroxy, mercapto, carboxyle ou acide sulfonique, ou forme – conjointement avec le groupe CO – l'extrémité terminale C d'un peptide de 2 à 10 aminoacides, les groupes amino présents dans $R^1$ pouvant éventuellement être bloqués par des groupes protecteurs connus,

X représente –O– ou –NQ–,

Q représente l'hydrogène, un reste alkyle en $C_1$ à $C_4$ portant éventuellement 1 ou 2 substituants OH, ou un reste (alkoxy en $C_1$ à $C_4$)-carbonyle,

Y désigne l'hydrogène, un halogène, un groupe cyano ou un reste carbamoyle,

$R^2$ désigne l'hydrogène ou un groupe acide sulfonique et

A représente un reste benzoxazole-2-yle, benzothiazole-2-yle, thiazole-5-yle, benzimidazole-2-yle, quinazole-4-one-2-yle, 5-phényl-1,3,4-thiadiazole-2-yle ou 4-pyridyle portant éventuellement 1 ou 2 substituants du groupe des substituants alkyle en $C_1$ à $C_4$, chloro, alkoxy en $C_1$ à $C_4$, alkylsulfonyle en $C_1$ à $C_4$, carboxyle, (alkoxy en $C_1$ à $C_4$) carbonyle, cyclohexyle, phényle ou acide sulfonique.

2. Composé suivant la revendication 1, caractérisé en ce que

$R^1$ est un reste alkyle ou alcényle en $C_1$ à $C_{18}$ non ramifié ou forme – conjointement avec le groupe CO – le reste d'extrémité terminale C d'un aminoacide naturel ou d'un peptide de 2 à 5 aminoacides.

3. Composés suivant la revendication 1 ou 2, caractérisés en ce que

Y représente CN et

A est un reste benzothiazole-2-yle ou benzoxazole-2-yle éventuellement substitué par un radical méthyle, éthyle, chloro, méthoxy, éthoxy ou sulfo.

4. Procédé de production de composés suivant les revendications 1 à 3, caractérisé en ce qu'on fait réagir avec un anhydride d'acide ou un halogénure d'acide des composés hydroxyliques de formule

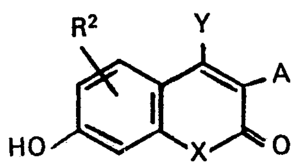

dans laquelle

X, Y, A et $R^2$ ont la définition mentionnée dans les revendications 1 à 3.

5. Procédé suivant la revendication 4, caractérisé en ce qu'on conduit la réaction avec un halogénure d'acide carboxylique en présence d'un accepteur d'acide inorganique ou organique.

6. Procédé de production de composés suivant les revendications 1 à 3, caractérisé en ce qu'on fait réagir des aldéhydes de formule

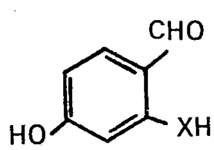

dans laquelle

X a la définition mentionnée dans les revendications 1 à 3 avec des composés de formule

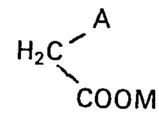

dans laquelle

A a la définition mentionnée dans les revendications 1 à 3

M est l'hydrogène ou un métal alcalin, dans un anhydride de formule

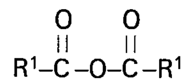

dans laquelle

$R^1$ a la définition mentionnée dans les revendications 1 à 3 comme solvant ou agent de condensation en présence d'un sel alcalin de l'acide carboxylique correspondant

$$R^1\text{–COOH}$$

dans les conditions de la synthèse de Perkin-Oglialoro par chauffage pendant plusieurs heures à des températures de 100 à 140 °C.

7. Compositions analytiques contenant des esters d'acides carboxyliques chromogènes ou fluorogènes pour la détection photométrique ou fluorimétrique d'hydrolases, notamment de carboxylestérases, caractérisées en ce qu'elles contiennent comme esters d'acides carboxyliques chromogènes ou fluorogènes des composés suivant les revendications 1 à 3.

8. Compositions analytiques suivant la revendication 7, caractérisées en ce qu'elles contiennent des générateurs micellaires anioniques ou cationiques destinés à améliorer l'hydrosolubilité des réactifs relatifs aux hydrolases.

9. Compositions analytiques suivant la revendication 7 ou 8, caractérisées en ce que les esters d'acides carboxyliques sont contenus dans une matière de support.

10. Méthode de détermination de l'activité en hydrolase d'un échantillon liquide, caractérisée en ce qu'on met en contact l'échantillon à analyser avec un composé suivant les revendications 1 à 3 ou une composition analytique suivant les revendications 7 à 9, et on mesure l'allure dans le temps de l'absorption de lumière ou de l'intensité de fluorescence.

**Claims**

Compounds of the general formula

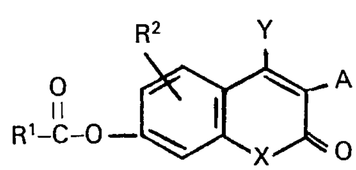

wherein

$R^1$ represents hydrogen, an optionally branched

or cyclic alkyl, alkenyl or alkinyl radical with 1 to 20 C atoms, which optionally carries 1 to 3 substituents from the group comprising amino, hydroxyl, mercapto, carboxyl and halogen and is optionally interrupted by 1 or 2 hetero atoms from the group comprising O, S and NQ, or – together with the CO group – represents the C-terminal radical of an amino acid, wherein $R_1$ represents a (hetero)arylalkyl radical with 6 to 20 C atoms, which optionally carries 1 to 3 substituents from the group comprising $C_1$–$C_4$-alkyl, halogen, $C_1$–$C_4$-alkoxy, amino, hydroxyl, mercapto, carboxyl and sulphonic acid or – together with the CO group – represents the C-terminal end of a peptide of 2 to 10 amino acids, it being possible for the amino groups present in $R^1$ optionally to be blocked by protective groups which are known per se,

X represents –O– or –NQ–,

Q represents hydrogen or a $C_1$–$C_4$-alkyl radical or $C_1$–$C_4$-alkoxycarbonyl radical which is optionally substituted by 1–2 OH groups

Y represents hydrogen, halogen, cyano or a carbamoyl radical,

$R^2$ represents hydrogen or a sulphonic acid group and

A represents a benzoxazol-2-yl, benzthiazol-2-yl, thiazol-5-yl, benzimidazol-2-yl, quinazol-4-on-2-yl, 5-phenyl-1,3,4-thiadiazol-2-yl or 4-pyridyl radical which is optionally substituted by 1–2 substituents from the group comprising $C_1$–$C_4$-alkyl, chlorine, $C_1$–$C_4$-alkoxy, $C_1$–$C_4$-alkylsulphonyl, carboxyl, $C_1$–$C_4$-alkoxycarbonyl, cyclohexyl, phenyl and sulphonic acid.

2. Compound according to Claim 1, characterized in that $R^1$ represents an unbranched $C_1$–$C_{18}$-alkyl or alkenyl radical or – together with the CO group – the C-terminal radical of a naturally occurring amino acid or of a peptide of 2 to 5 amino acids.

3. Compounds according to Claim 1 or 2, characterized in that

Y represents CN and

A represents a benzthiazol-2-yl or benzoxazol-2-yl radical which is optionally substituted by methyl, ethyl, chlorine, methoxy, ethoxy or sulpho.

4. Process for the preparation of compounds according to Claims 1 to 3, characterized in that hydroxyl compounds of the formula

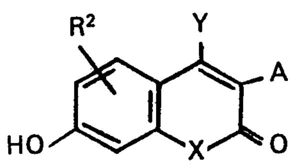

wherein

X, Y, A and $R_2$ have the meaning mentioned in Claims 1–3, are reacted with an acid anhydride or acid halide.

5. Process according to Claim 4, characterized in that the reaction is carried out with a carboxylic acid halide in the presence of an inorganic or organic acid-trapping agent.

6. Process for the preparation of compounds according to Claims 1 to 3, characterized in that aldehydes of the formula

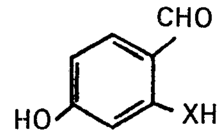

wherein

X has the meaning given in Claims 1–3, are reacted with compounds of the formula

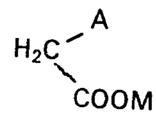

wherein

A has the meaning given in Claims 1 to 3, and

M represents hydrogen or an alkali metal, in an anhydride of the formula

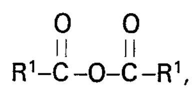

wherein

$R^1$ has the meaning given in Claims 1 to 3 as the solvent or condensing agent, in the presence of an alkali metal salt of the corresponding carboxylic acid

$$R^1\text{–COOH}$$

under the conditions of the Perkin-Oglialoro synthesis by heating to temperatures of 100–140 °C for several hours.

7. Testing agents, containing chromogenic or fluorogenic carboxylic acid esters, for the photometric or fluorimetric detection of hydrolases, especially carboxylesterases, characterized in that they contain compounds according to Claim 1 to 3 as the chromogenic or fluorogenic carboxylic acid esters.

8. Testing agents according to Claim 7, characterized in that they contain anionic or cationic micell-forming agents to improve the water-solubility of the hydrolase reagents.

9. Testing agents according to Claim 7 or 8, characterized in that the carboxylic acid esters are contained in a carrier material.

10. Process for the determination of hydrolase activity in a liquid sample, characterized in that the sample to be investigated is brought into contact with a compound according to Claim 1 to 3 or a testing agent according to Claim 7 to 9 and the course of the light absorption or fluorescence intensity with respect to time is measured.